# FASCICULE DE BREVET EUROPEEN

(11) **EP 2 906 704 B1**
(45) Date de publication et mention de la délivrance du brevet: **27.12.2017**
(21) Numéro de dépôt: 13799108.9
(22) Date de dépôt: 08.10.2013
(51) Int. Cl.: C12N 15/86, C12N 7/00, A61K 39/00, A61K 39/12, A61K 39/205

(54) **NOVIRHABDOVIRUS RECOMBINANT UTILISABLE COMME VECTEUR D'ANTIGÈNES**
REKOMBINANTES NOVIRHABDOVIRUS ALS ANTIGENVEKTOR
RECOMBINANT NOVIRHABDOVIRUS USABLE AS AN ANTIGEN VECTOR

(30) Priorité: 15.10.2012 FR 1259815
(43) Date de publication de la demande: 19.08.2015
(73) Titulaire: Institut National de la Recherche Agronomique (INRA), 75007 Paris (FR)
(72) Inventeur: BREMONT, Michel, 92310 Sevres (FR); NZONZA, Angella, F-77310 Ponthierry (FR)
(74) Mandataire: Gevers & Orès
(86) Numéro de dépôt international: PCT/IB2013/059207
(87) Numéro de publication internationale: WO 2014/060905

(56) Documents cités:
- WO-A1-03/097090
- WO-A1-2004/026338
- WO-A2-2007/144773
- SCHNELL ET AL: "foreign glycoproteins expressed from recombinant vesicular stomatitis viruses are incorporated efficientl into virus particles", PROCEEDINGS OF THE NATIONAL ACADEMY OF SCIENCES, NATIONAL ACADEMY OF SCIENCES, US, vol. 93, octobre 1996 (1996-10), pages 11359-11365, XP002100328, ISSN: 0027-8424, DOI: 10.1073/PNAS.93.21.11359
- L. D. SCHLEHUBER ET AL: "Prediction and Identification of a Permissive Epitope Insertion Site in the Vesicular Stomatitis Virus Glycoprotein", JOURNAL OF VIROLOGY, vol. 78, no. 10, 15 mai 2004 (2004-05-15), pages 5079-5087, XP055070289, ISSN: 0022-538X, DOI: 10.1128/JVI.78.10.5079-5087.2004
- DESMEZIERES E ET AL: "Lyssavirus glycoproteins expressing immunologically potent foreign B cell d cytotoxic T lymphocyte epitopes as prototypes for multivalent ccines", JOURNAL OF GENERAL VIROLOGY, SOCIETY FOR GENERAL MICROBIOLOGY, SPENCERS WOOD, GB, vol. 80, no. PART 9, septembre 1999 (1999-09), pages 2343-2351, XP002145723, ISSN: 0022-1317

## Description

La présente invention concerne des novirhabdovirus recombinants utilisables comme vecteurs d'antigènes, et notamment de protéines antigéniques non-glycosylées, pour induire une réponse immune contre lesdits antigènes.

Les novirhabdovirus sont des virus à ARN négatif de la famille des rhabdovirus.

Les rhabdovirus sont des virus enveloppés. Le virion comprend une nucléocapside à symétrie hélicoïdale, résultant de l'assemblage de molécules de protéine N autour du brin d'ARN génomique, et à laquelle sont associées des molécules des protéines L et P. L'enveloppe qui recouvre la nucléocapside est constituée d'une double couche lipidique d'origine cellulaire, dont la face interne est tapissée par la protéine M, et dans laquelle sont insérés des spicules formés de trimères de glycoprotéine G, intervenant dans l'attachement du virus à la cellule infectée, et sa fusion avec la membrane cellulaire. La glycoprotéine G des rhabdovirus est, sous sa forme mature, un polypeptide d'environ 500 acides aminés, constitué d'un ectodomaine d'environ 435 à environ 450 acides aminés en N-terminal, suivi d'un domaine transmembranaire de 21 à 23 acides aminés, et d'un domaine intravirion (également dénommé domaine intracytoplasmique), d'environ 25 à environ 45 acides aminés. Elle est synthétisée dans le cytoplasme des cellules infectées sous forme d'un précurseur de 505 à 525 acides aminés, dont le peptide signal est ensuite clivé dans le réticulum endoplasmique.

Le genre *Novirhabdovirus* comprend différentes espèces pathogènes des animaux aquatiques, notamment des poissons.

L'espèce type du genre est le virus de la nécrose hématopoïétique infectieuse (VNHI) qui est l'agent étiologique d'une maladie grave chez plusieurs espèces de salmonidés. D'autres espèces du genre sont le rhabdovirus hirame (HIRRV), le virus de la septicémie hémorragique virale (VSHV) et le rhabdovirus du poisson serpent (SHRV pour : snakehead rhabdovirus).

La structure du génome des novirhabdovirus est proche de celle des rhabdovirus de mammifères, mais se différencie de celle-ci par la présence d'un gène supplémentaire, codant pour une protéine non-structurale, dénommée protéine NV (pour « non-virion »).

Le génome des novirhabdovirus comprend donc six gènes, dont l'organisation peut être schématisée comme suit :

3'-N-P-M-G-NV-L-5'

N représente le gène codant pour la nucléoprotéine associée à l'ARN viral, P représente le gène codant pour la phosphoprotéine associée à la polymérase virale, M représente le gène codant pour la protéine de matrice, G représente le gène codant pour la glycoprotéine d'enveloppe G (également dénommée protéine de spicule), NV représente le gène codant pour la protéine NV, et L représente le gène codant pour l'ARN polymérase virale ARN dépendante.

Ces gènes sont séparés par des régions intergéniques: chacune d'elles comprend un signal d'initiation de la transcription du gène situé en aval de celui-ci, et un signal de terminaison de la transcription et de polyadénylation, , ce qui permet la transcription des gènes en ARNm individuels.

Il a été montré que les novirhabdovirus pouvaient être utilisés en tant que vecteurs d'expression de gènes hétérologues, et notamment d'antigènes vaccinaux.

Des systèmes de génétique inverse, similaires à ceux existant pour les rhabdovirus de mammifères sont en effet disponibles pour produire des novirhabdovirus recombinants. Classiquement, ces systèmes sont basés sur la co-transfection d'une cellule-hôte exprimant une ARN polymérase (généralement l'ARN polymérase T7) par l'ADN complémentaire (ADNc) du génome viral complet, et des vecteurs d'expression codant pour les protéines N, P, et L du complexe réplicatif viral.

En vue d'utiliser ces novirhabdovirus recombinants comme vecteurs viraux, différentes approches ont été utilisées pour y introduire des gènes étrangers.

Une première approche repose sur le remplacement d'un gène endogène de novirhabdovirus par un gène hétérologue. Par exemple, il a été ainsi montré, chez le VNHI, le VSHV et le SHRV, que le gène de la glycoprotéine G pouvait être remplacé par celui d'un autre novirhabdovirus, et que le gène NV pouvait être délété et remplacé par un gène étranger (BIACCHESI et al., J Virol, 74, 11247-53, 2000; BIACCHESI et al., J Virol, 76, 2881-9, 2002; BIACCHESI et al., J Virol, 84, 10038-50, 2010; ALONSO et al., Journal of Virology, 78, 5875-82, 2004 ; Demande PCT WO 2003/097090).

Une seconde approche consiste à insérer un ou plusieurs gènes dans une ou plusieurs des régions intergéniques du génome viral, sous forme d'une ou plusieurs unité(s) de transcription supplémentaire(s), chaque unité de transcription comprenant un signal d'initiation de la transcription, suivi de la séquence codante de la protéine d'intérêt à exprimer, cette séquence codante étant elle même suivie d'un signal de terminaison de la transcription/polyadénylation.

Cette approche a été utilisée notamment chez le VNHI et le VSHV, pour exprimer *in vivo* différents gènes rapporteurs (HARMACHE et al., J Virol, 80, 3655-9, 2006; BIACCHESI et al., J Virol, 84, 10038-50, 2010). Elle a également permis d'exprimer des antigènes vaccinaux ; la Demande PCT WO 2007/144773 décrit la construction de VNHI recombinants comprenant de 1 à 3 inserts intergéniques exprimant des antigènes de divers virus pathogènes des salmonidés, et montre que ces VNHI recombinants sont capables de se multiplier normalement en cultures cellulaire, et peuvent induire une réponse immune protectrice contre les virus concernés lorsqu'ils sont utilisés pour immuniser des truitelles. En outre, lorsque l'un de ces VNHI recombinants exprimant un antigène hétérologue est injecté *in vivo* chez la souris, il est incapable de s'y répliquer, mais induit cependant une réponse anticorps dirigée notamment contre l'antigène hétérologue.

Les novirhabdovirus présentent donc de nombreux avantages en tant que vecteurs d'expression d'antigènes hétérologues.

Pour améliorer encore l'efficacité de la réponse antigénique dirigée contre des antigènes hétérologues exprimés par des novirhabdovirus, il est souhaitable que ces antigènes soient exposés à la surface des particules virales. Toutefois, les Inventeurs ont constaté que dans le cas des novirhabdovirus recombinants précédemment décrits, seuls certains antigènes, de nature glycoprotéique et capables de s'insérer naturellement dans des membranes cellulaires, étaient présentés en surface des particules virales.

En cherchant à remédier à cet inconvénient, les Inventeurs ont constaté que lorsqu'on insérait dans le génome d'un novirhabdovirus un gène codant pour un polypeptide chimérique contenant la séquence d'un antigène d'intérêt flanquée à son extrémité N-terminale de la séquence du peptide signal d'une protéine G de novirhabdovirus, et à son extrémité C-terminale de la séquence du domaine transmembranaire d'une protéine G de novirhabdovirus, l'expression de ce gène chimérique aboutissait à l'incorporation de la forme mature de son produit de traduction dans l'enveloppe virale, sans que cette incorporation interfère avec l'assemblage du virion, ni avec sa capacité à infecter des cellules en culture et à s'y répliquer.

La présente invention a pour objet un novirhabdovirus recombinant contenant dans son génome, outre les gènes codant pour les protéines N, P, M, G et L endogènes dudit novirhabdovirus, un gène exogène codant pour une protéine chimérique, ladite protéine chimérique étant caractérisée en ce qu'elle comprend la séquence d'une protéine antigénique d'intérêt, ladite protéine antigénique d'intérêt n'étant pas une glycoprotéine, fusionnée à son extrémité N terminale avec le peptide signal de la protéine G d'un rhabdovirus et à son extrémité C-terminale avec un fragment de séquence d'une protéine G de rhabdovirus, ledit fragment comprenant le domaine transmembranaire de ladite protéine G de rhabdovirus ou une portion de celui-ci comprenant au moins 15 acides aminés C-terminaux consécutifs dudit domaine, caractérisé en ce qu'il comprend en outre, à la suite du domaine transmembranaire ou de la portion de celui-ci, le domaine intravirion d'une protéine G de rhabdovirus, ou une portion dudit domaine intravirion comprenant au moins 3 acides aminés N-terminaux consécutifs dudit domaine

La portion dudit domaine transmembranaire de ladite protéine G de rhabdovirus comprend, de préférence au moins 16,
et par ordre de préférence croissante au moins 17, 18, 19, ou 20 acides aminés consécutifs dudit domaine. On définit ici comme domaine transmembranaire de la protéine G d'un rhabdovirus, la région de la protéine G située entre l'ectodomaine et le domaine intravirion de ladite protéine. Le domaine transmembranaire peut être facilement localisé par l'homme du métier dans la séquence d'une protéine G, par exemple à partir des annotations figurant dans des bases de données, ou le cas échéant à l'aide d'un logiciel de prédiction de domaines protéiques, tel que TMHMM (http://www.cbs.dtu.dk/services/TMHMM) ou InterProScan (http://www.ebi.ac.uk/Tools/pfa/iprscan/).

A titre d'exemples non-limitatifs, selon les indications fournies par la base de données Uniprot/Swissprot, le domaine transmembranaire de la protéine G correspond chez les novirhabdovirus, aux acides aminés 462-482 de la séquence du précurseur de la protéine G dans le cas du VHSV, du VNHI et du rhabdovirus hirame, et aux acides aminés 464-486 dans le cas du SHRV. Chez des rhabdovirus autres que les novirhabdovirus, ce domaine transmembranaire correspond aux acides aminés 460-480 de la séquence du précurseur de la protéine G dans le cas du virus de la rage (Lyssavirus), aux acides aminés 468-488 dans le cas du virus de la stomatite vésiculaire (Vesiculovirus), et aux acides aminés 462-482 dans le cas du virus de la virémie printanière de la carpe (Vesiculovirus).

Avantageusement ledit domaine transmembranaire est choisi parmi ceux des protéines G de lyssavirus, de vesiculovirus, et de novirhabdovirus. De manière tout à fait préférée, il s'agit du domaine transmembranaire de la protéine G d'un novirhabdovirus.

Généralement, pour des raisons de commodité de construction du
gène chimérique, ledit domaine intravirion sera issu de la protéine G du même rhabdovirus que le domaine transmembranaire ; il est toutefois possible d'associer un domaine transmembranaire et un domaine intravirion (ou leurs portions) issus de rhabdovirus différents. La portion du domaine intravirioncomprend avantageusement au moins 4, et par ordre de préférence croissante au moins 5, 6, 7, 8, 9, ou 10 acides aminés consécutifs dudit domaine. Bien qu'il soit préféré que l'extrémité C-terminale de la protéine antigènique d'intérêt soit directement fusionnée à l'extrémité N-terminale du domaine transmembranaire, il est toutefois envisageable, dans certains cas, que quelques acides aminés C-terminaux de l'ectodomaine (généralement moins de 20, de préférence moins de 10, et avantageusement moins de 5) soient présents entre la protéine antigènique d'intérêt et le domaine transmembranaire.

Le peptide signal de la protéine G d'un rhabdovirus peut être tout peptide permettant l'adressage de la protéine chimérique dans le réticulum endoplasmique de la cellule infectée par le novirhabdovirus, suivi du clivage dudit peptide signal. Un grand nombre de peptides signaux utilisables dans ce but sont connus en eux mêmes de l'homme du métier. On peut le cas échéant utiliser le peptide signal endogène de protéine antigénique d'intérêt que l'on souhaite exprimer, si elle en possède un. Avantageusement, on peut utiliser le peptide signal de la protéine G d'un novirhabdovirus ; il n'est pas indispensable que ce peptide signal soit issu de la protéine G du même virus que le domaine transmembranaire et/ou que le domaine intravirion.

La protéine ou le fragment antigénique d'intérêt sont de nature non-glycoprotéique, c'est-à-dire qu'il s'agit de polypeptides qui ne sont pas glycosylés lorsqu'ils sont exprimés dans des cellules animales.

Il peut s'agir notamment d'un antigène dérivé d'un pathogène viral, bactérien, fongique, d'un parasite protozoaire, ou d'un antigène tumoral, ou bien d'une protéine recombinante associant divers fragments antigéniques, issus d'un même antigène ou d'antigènes différents.

La taille de cette protéine antigénique peut varier de quelques acides aminés à quelques centaines d'acides aminés ; de préférence elle sera de 100 à 600 acides aminés, et de manière tout à fait préférée de 300 à 600 acides aminés.

A titre d'exemple non-limitatif de protéine antigénique de nature non-glycoprotéique, on citera le domaine III de la protéine E des flavivirus. Bien que la protéine E entière soit une glycoprotéine, son domaine III ne contient aucun site de glycosylation.

Un novirhabdovirus recombinant conforme à l'invention peut être obtenu à partir de n'importe quel novirhabdovirus, notamment VNHI, VSHV, HIRRV, ou SHRV. Des novirhabdovirus préférés sont VNHI et VSHV.

Selon un premier mode de réalisation d'un novirhabdovirus recombinant conforme à l'invention, le gène codant pour la protéine chimérique est inséré en remplacement du gène NV endogène dudit novirhabdovirus. Des novirhabdovirus recombinants conformes à ce premier mode de réalisation peuvent être construits comme décrit par exemple par BIACCHESI et al., (2000, 2002, 2010, précités), ALONSO et al., (2004 précité) ou dans la Demande PCT WO 2003/097090.

Selon un deuxième mode de réalisation d'un novirhabdovirus recombinant conforme à l'invention, il conserve le gène NV endogène, et le gène codant pour la protéine chimérique est inséré dans une unité de transcription additionnelle placée dans une région intergénique du génome viral. Des novirhabdovirus recombinants conformes à ce deuxième mode de réalisation peuvent être construits comme décrit par exemple par BIACCHESI et al., (2010, précité), HARMACHE et al., (2006, précité) ou dans la Demande PCT WO 2007/144773.

Conformément à ce deuxième mode de réalisation, ledit novirhabdovirus peut contenir plusieurs unités de transcription additionnelles, dont chacune contient un gène exogène codant pour une protéine chimérique. De préférence ledit novirhabdovirus contient deux unités de transcription additionnelle, et de manière tout à fait préférée trois unités de transcription additionnelles. Avantageusement lesdites protéines chimériques différent entre elles au moins par la nature de l'antigène d'intérêt, et optionnellement par celle du peptide signal et/ou du domaine transmembranaire et/ou du domaine intravirion.

La présente invention a également pour objet des constructions d'ADN recombinant permettant l'obtention d'un novirhabdovirus conforme à l'invention.

Dans ce cadre, la présente invention englobe notamment l'ADNc du génome d'un novirhabdovirus recombinant conforme à l'invention, ainsi que tout vecteur recombinant comprenant ledit ADNc.

La présente invention a également pour objet les utilisations d'un novirhabdovirus recombinant conforme à l'invention pour induire une réponse immune humorale et/ou cellulaire contre la protéine antigénique d'intérêt exprimée par ledit novirhabdovirus.

Notamment, la présente invention a pour objet un novirhabdovirus recombinant conforme à l'invention pour l'utilisation comme médicament, et notamment comme vaccin.

Les vaccins contenant un novirhabdovirus recombinant conforme à l'invention peuvent être utilisés chez les poissons, et en particulier les salmonidés, tels que les truites et les saumons d'élevage, selon les méthodes décrites dans la Demande PCT WO 2003/097090 ou dans la Demande PCT WO 2007/144773.

Les novirhabdovirus recombinants conformes à l'invention, et en particulier ceux qui ne se répliquent qu'à basse température comme VNHI et VHSV sont utilisables pour l'obtention de vaccins, non seulement chez les poissons, mais aussi chez d'autres animaux, incluant les oiseaux et les mammifères, et notamment les ovins, les bovins, les porcins, les équins, les canins, les félins, et les primates, en particulier l'homme. En effet, bien que les novirhabdovirus soient incapables de se répliquer chez un animal homéotherme, ils sont capables d'induire une forte réponse immune contre un antigène hétérologue d'intérêt présenté à la surface de la particule virale.

Dans ce cadre, les novirhabdovirus conformes à l'invention peuvent être utilisés comme vaccins anti-viral, anti-bactérien, anti-fongique, ou anti-tumoral, selon la nature de l'antigène d'intérêt choisi.

Ces vaccins peuvent être formulés pour une utilisation par voie parentérale, par exemple par voie intradermique, intramusculaire, ou sous-cutanée, par voie orale, ou par voie muqueuse, par exemple intra-nasale. La quantité de virus recombinants par dose vaccinale est choisie de manière à permettre un niveau d'expression de la protéine antigénique d'intérêt suffisant pour induire une réponse immunitaire contre cette protéine. Elle peut être déterminée par l'homme du métier notamment en fonction de la nature de ladite protéine antigénique, de l'espèce et de l'âge du sujet à vacciner, du type de réponse immunitaire (cellulaire ou humorale) que l'on souhaite privilégier.

La présente invention a également pour objet une méthode non-thérapeutique pour la production d'anticorps dirigés contre la protéine antigénique d'intérêt, ladite méthode comprenant l'immunisation d'un animal non-humain avec un novirhabdovirus recombinant comprenant ladite protéine antignéique d'intérêt et conforme à l'invention, et la récupération de son sérum (pour la production d'anticorps polyclonaux), ou de ses cellules lymphocytaires (pour la production d'anticorps monoclonaux).

La présente Invention sera mieux comprise à l'aide du complément de description qui va suivre, qui se réfère à des exemples non-limitatifs de construction d'un novirhabdovirus recombinant conforme à l'invention.

### EXEMPLE 1 : CONSTRUCTION D'UN NOVIRHABDOVIRUS RECOMBINANT CONTENANT UN GENE CODANT POUR UNE PROTEINE CHIMERIQUE COMPRENANT DOMAINE III DE LA GLYCOPROTEINE E DU WEST NILE VIRUS (WNV).

Les constructions ont été effectuées à partir du plasmide pVHSV, décrit par BIACCHESI et al. (2010, précité). Ce plasmide contient l'ADNc complet du génome d'un virus VHSV (souche 23-75, GenBankFN665788), cloné en aval du promoteur de l'ARN polymérase du phage T7 et en amont d'une séquence ribozyme du virus de l'hépatite δ et du terminateur de transcription de l'ARN polymérase du phage T7, dans le vecteur pBlueScript SK (STRATAGENE).

Le plasmide pVHSV contient un site de restriction unique *PsiI* situé dans la région intergénique entre les gènes N et P. Ce site a été utilisé pour insérer une unité de transcription additionnelle, contenant une séquence codant pour une protéine de fusion constituée par la séquence du domaine III de la glycoprotéine E du West Nile Virus (GenBank AF481864) précédée par le peptide signal de la protéine G de VHSV (souche 23-75, GenBank CBJ23832.1.), et suivie par les qui contient un site *SpeI* (ACTAGT) et un site *NheI* (GCTAGC) et DIIISHVTMR :
5'-GGCCCCTCCCACAACCCCCATCCCAGATAACGCTCCTTTGAGGGTGGTTGTAAAGG-3' (SEQ ID NO: 2).

Une seconde amplification PCR a été effectuée à partir de l'ADNc de VHSV, en utilisant les amorces suivantes :
DIIITMSHVF :
   5'-CCTTTACAACCACCCTCAAAGGAGCGTTATCTGGGATGGGGGTTGTGGGAGGGGCC-3' (SEQ ID NO: 3), et
SHVTMR :
   5'-TACGTATCAGACCGTCTGACTTCTAGAGAACTGC-3' (SEQ ID NO: 4), qui contient un site *SnaBI* (TACGTA)

Les deux produits d'amplification ont été mélangés, et une troisième amplification PCR a été effectuée à partir du mélange, en utilisant les amorces :
SPSHVF :
   5'-ACTAGTATGGACACCACGATCACCACTCCGC-3' (SEQ ID NO: 5), qui contient un site *SpeI* (ACTAGT), et SHVTMR (SEQ ID NO: 4).

Le produit de cette troisième amplification (SPg-DIII-TMg), qui contient la séquence codant pour le peptide signal de la protéine G de VHSV, en phase de lecture avec celle codant pour le domaine III de la glycoprotéine E du West Nile Virus, et celle codant pour les 42 acides aminés C-terminaux de la protéine G de VHSV a été cloné dans un vecteur pJet1.2 (FERMENTAS). L'insert SPg-DIII-TMg a été excisé de ce vecteur par digestion *SpeI*/*SnaBI* et cloné, à la place du gène tdTomato, dans le plasmide pVSHV-dTomato (décrit par BIACCHESI et al., 2010, précité) préalablement digéré par *SpeI*/*SnaBI,* pour obtenir la construction finale pSHV-SPg-DIII-TMg.

### Production des novirhabdovirus recombinants :

Trois plasmides d'expression comportant respectivement les gènes codant pour la nucléoprotéine N, la phosphoprotéine P, et l'ARN polymérase ARN dépendante L de VHSV ont été construits, comme décrit par BIACCHESI et al. (2010, publication précitée). Ces constructions sont respectivement appelées pT7-N, pT7-P et pT7-L.

Le plasmide pVHSV ou le plasmide pVHSV-SPg-DIII-TMg, à une dose de 1 µg, et les 3 plasmides, pT7-N, pT7-P, pT7-L, à des doses respectives de 0,25 µg, 0,2 µg, 0,2 µg) sont introduits par transfection en présence de Lipofectamine (GIBCO-BRL) dans des cellules EPC préalablement infectées par un virus de la vaccine recombinant exprimant l'ARN polymérase du phage T7 (vTF7-3, FUERST et al. Proc. Natl. Acad. Sci. USA, 92, 4477-4481, 1986).

Après transfection, les cellules sont incubées pendant 5 heures à 37°C puis lavées avec du milieu de culture MEM (sans sérum) et incubées pendant 7 jours à 14 °C en milieu de culture MEM contenant 2% de sérum foetal de veau. Les cellules et le surnageant sont congelés/décongelés, et clarifiés par centrifugation pendant 10 minutes à 10000 tours/min. Le surnageant est utilisé à la dilution 1/10 pour infecter un tapis de cellules EPC (Epithelioma Papulosum Cyprini, dérivées de cellules épithéliales de carpe). Les virus sont produits dans le surnageant 3-4 jours postinfection.

Les virus obtenus sont respectivement dénommés rVHSV, dans le cas du virus possédant le génome du virus sauvage, et rVHSV-SPg-DIII-TMg dans le cas du virus contenant le gène codant pour la protéine de fusion.

Des stocks viraux de chacun des virus produits ont été constitués par passages successifs en culture cellulaire du surnageant prélevé 7 jours après transfection (surnageant PO) sur des cellules EPC. Les cellules sont infectées à une multiplicité d'infection (m.i) de 1. Après 3 passages, les surnageants ont été prélevés à différents temps postinfection, et titrés par dilution limite pour établir une courbe de croissance.

Les courbes de croissance établies pour les virus rVHSV et rVHSV-SPg-DIII-TMg, montrent que le virus rVHSV-SPg-DIII-TMg se multiplie en culture cellulaire aussi bien que le virus rVHSV.

L'expression du domaine III de la glycoprotéine E du West Nile Virus dans les cellules infectées par rVHSV-SPg-DIII-TMg a été vérifiée à 2 jours post infection, par un test d'immunofluorescence indirecte à l'aide d'un anticorps monoclonal anti-DIII, sur cellules infectées vivantes ou fixées à l'alcool/acétone.

### EXEMPLE 2 : EXPRESSION DU DOMAINE III DE LA GLYCOPROTÉINE E DU WEST NILE VIRUS À LA SURFACE DU NOVIRHABDOVIRUS.

Des cellules EPC ont été infectées comme décrit dans l'Exemple 1 ci-dessus, par le virus rVHSV ou par le virus rVHSV-SPg-DIII-TMg.

Trois jours après l'infection, le surnageant de culture a été récupéré, et les virus purifiés sur gradient de sucrose à partir de ce surnageant.

Les protéines virales ont été séparées par électrophorèse SDS-PAGE et visualisées après coloration au bleu de Coomassie, ou après transfert de Western et incubation avec un anticorps monoclonal dirigé contre le domaine III de la glycoprotéine E du WNV.

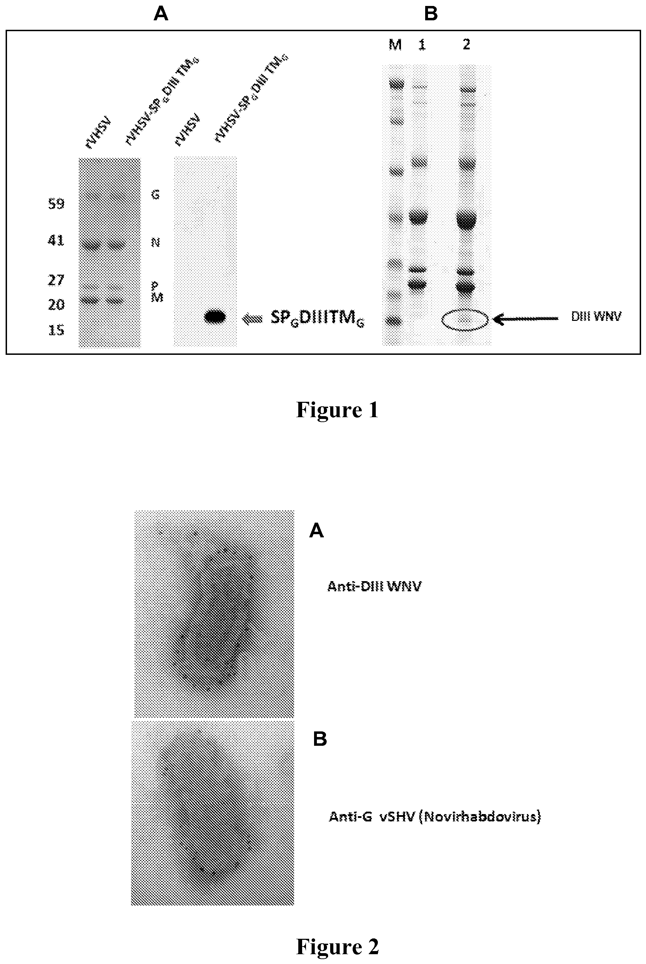

Les résultats sont illustrés par la Figure 1.

Légende de la Figure 1 :
A : Panneau de gauche :Gel SDS PAGE coloré au bleu de Coomassie des virus rVHSV et rVHSV-SPg DIII TMg purifiés sur gradient de sucrose avant transfert pour Western blot ;
   Panneau de droite : transfert de Western avec l'anticorps monoclonal dirigé contre le domaine III de la glycoprotéine E du WNV ;
B : Gels SDS PAGE de virus purifié coloré que Bleu de Coomassie.
M : Marqueur de poids moléculaire,
1 : rVHSV
2 : rVHSV-SPg-DIII-TMg.

Ces résultats montrent que le domaine III de la glycoprotéine E du WNV est fortement exprimé dans les particules du virus rVHSV-SPg-DIII-TMg.

Les particules virales rVHSV-SPg-DIII-TMg purifiées ont également été observées en microscopie électronique après immunomarquage à l'or colloïdal en utilisant soit un anticorps dirigé contre le domaine III de la glycoprotéine E du WNV, soit un anticorps dirigé contre la glycoprotéine G de VSHV.

Les résultats sont illustrés par la Figure 2. Les points noirs à la surface des particules virales indiquent la présence du domaine III de la glycoprotéine E du WNV (Figure 2A), ainsi que de la glycoprotéine G de VSHV (Figure 2B) à la surface des virus rVHSV-SPg-DIII-TMg.

Cela montre que l'antigène d'intérêt est très fortement et très efficacement exprimé à la surface des particules virales.

## Revendications

1. Novirhabdovirus recombinant dont le génome contient, outre les gènes codant pour les protéines N, P, M, G et L endogènes dudit novirhabdovirus, un gène exogène codant pour une protéine chimérique, ladite protéine chimérique étant **caractérisée en ce qu'**elle comprend la séquence d'une protéine antigénique d'intérêt, ladite protéine antigénique d'intérêt n'étant pas une glycoprotéine, fusionnée à son extrémité N terminale avec le peptide signal de la protéine G d'un rhabdovirus et à son extrémité C-terminale avec un fragment de séquence d'une protéine G de rhabdovirus, ledit fragment comprenant le domaine transmembranaire de ladite protéine G de rhabdovirus ou une portion de celui-ci comprenant au moins 15 acides aminés C-terminaux consécutifs dudit domaine, **caractérisé en ce qu'**il comprend en outre, à la suite du domaine transmembranaire ou de la portion de celui-ci, le domaine intravirion d'une protéine G de rhabdovirus, ou une portion dudit domaine intravirion comprenant au moins 3 acides aminés N-terminaux consécutifs dudit domaine.

2. Novirhabdovirus recombinant selon la revendication 1, **caractérisé en ce que** ledit domaine transmembranaire et/ou ledit domaine intravirion est (sont) choisi(s) parmi ceux des protéines G de lyssavirus, de vesiculovirus, et de novirhabdovirus.

3. Novirhabdovirus recombinant selon une quelconque des revendications 1 ou 2, **caractérisé en ce qu'**il est dépourvu du gène NV endogène, et **en ce que** le gène codant pour la protéine chimérique est inséré en remplacement dudit gène NV.

4. Novirhabdovirus recombinant selon une quelconque des revendications 1 à 3, **caractérisé en ce qu'**il contient le gène NV endogène, et **en ce que** le gène codant pour la protéine chimérique est inséré dans une unité de transcription additionnelle placée dans une région intergénique du génome viral.

5. Novirhabdovirus recombinant selon la revendication 4 **caractérisé en ce qu'**il contient au moins deux unités de transcription additionnelles dont chacune contient un gène exogène codant pour une protéine chimérique telle que définie dans une des revendications 1 ou 2.

6. Novirhabdovirus recombinant selon une quelconque des revendications 1 à 5, **caractérisé en ce qu'**il est choisi parmi un virus de la nécrose hématopoïétique infectieuse, et un virus de la septicémie hémorragique virale.

7. Molécule d'ADNc du génome d'un novirhabdovirus recombinant selon une quelconque des revendications 1 à 6.

8. Novirhabdovirus recombinant selon une quelconque des revendications 1 à 6 pour l'utilisation comme vaccin induisant une réponse immune dirigée contre l'antigène d'intérêt exprimé par ledit novirhabdovirus.

9. Novirhabdovirus recombinant selon une quelconque des revendications 1 à 6 pour l'utilisation comme vaccin selon la revendication 8, **caractérisé en ce que** ledit vaccin est choisi parmi un vaccin anti-viral, un vaccin anti-bactérien, un vaccin anti-fongique, un vaccin anti-parasitaire, ou un vaccin anti-tumoral.

10. Méthode non-thérapeutique pour la production d'anticorps dirigés contre une protéine antigénique d'intérêt, ladite méthode comprenant l'immunisation d'un animal non-humain avec un novirhabdovirus selon une quelconque des revendications 1 à 6 comprenant ladite protéine antigénique d'intérêt.

## Patentansprüche

1. Rekombinantes Novirhabdovirus, dessen Genom neben den für die endogenen Proteine N, P, M, G und L des Novirhabdovirus codierenden Genen ein exogenes Gen enthält, das für ein chimärisches Protein codiert, wobei das chimärische Protein **dadurch gekennzeichnet ist, dass** es die Sequenz eines antigenen Proteins von Interesse umfasst, wobei das antigene Protein von Interesse kein Glykoprotein ist, die an ihrem N-Terminus mit dem Signalpeptid des Proteins G eines Rhabdovirus und an ihrem C-Terminus mit einem Sequenzfragment eines Rhabdovirus-Proteins G fusioniert ist, wobei das Fragment die Transmembrandomäne des Rhabdovirus-Proteins G oder einen Abschnitt davon umfasst, der mindestens 15 aufeinanderfolgende C-Terminus-Aminosäuren der Domäne umfasst, **dadurch gekennzeichnet, dass** es ferner im Anschluss an die Transmembrandomäne oder den Abschnitt davon die Intraviriondömäne eines Rhabdovirus-Proteins G oder einen Abschnitt der Intraviriondömäne umfasst, der mindestens 3 aufeinanderfolgende N-Terminus-Aminosäuren der Domäne umfasst.

2. Rekombinantes Novirhabdovirus nach Anspruch 1, **dadurch gekennzeichnet, dass** die Transmembrandomäne und/oder die Intraviriondömäne unter denjenigen der Proteine G des Lyssavirus, des Vesiculovirus und des Novirhabdovirus ausgewählt ist (sind).

3. Rekombinantes Novirhabdovirus nach einem der Ansprüche 1 oder 2, **dadurch gekennzeichnet, dass** es kein endogenes Gen NV aufweist, und dadurch, dass das codierende Gen für das chimärische Protein als Austausch des Gens NV eingesetzt ist.

4. Rekombinantes Novirhabdovirus nach einem der Ansprüche 1 bis 3, **dadurch gekennzeichnet, dass** es das endogene Gen NV enthält, und dadurch, dass das codierende Gen für das chimärische Protein in eine zusätzliche Transkriptionseinheit eingesetzt ist, die in einer intergenischen Region des viralen Genoms platziert ist.

5. Rekombinantes Novirhabdovirus nach Anspruch 4, **dadurch gekennzeichnet, dass** es mindestens zwei zusätzliche Transkriptionseinheiten enthält, von denen jede ein codierendes exogenes Gen für ein chimärisches Protein nach einem der Ansprüche 1 oder 2 umfasst.

6. Rekombinantes Novirhabdovirus nach einem der Ansprüche 1 bis 5, **dadurch gekennzeichnet, dass** es unter einem Virus der infektiösen hämatopoetischen Nekrose und einem Virus der viralen hämorrhagischen Septikämie ausgewählt ist.

7. cDNA-Molekül des Genoms eines rekombinanten Novirhabdovirus nach einem der Ansprüche 1 bis 6.

8. Rekombinantes Novirhabdovirus nach einem der Ansprüche 1 bis 6 zur Verwendung als Impfstoff, der eine Immunantwort induziert, die gegen das durch den Novirhabdovirus exprimierte Antigen von Interesse gerichtet ist.

9. Rekombinantes Novirhabdovirus nach einem der Ansprüche 1 bis 6 zur Verwendung als Impfstoff nach Anspruch 8, **dadurch gekennzeichnet, dass** der Impfstoff unter einem antiviralen Impfstoff, einem antibakteriellen Impfstoff, einem fungiziden Impfstoff, einem antiparasitären Impfstoff oder einem antitumoralen Impfstoff ausgewählt ist.

10. Nicht-therapeutisches Verfahren zur Produktion von Antikörpern, die gegen ein antigenes Protein von Interesse gerichtet sind, wobei das Verfahren die Immunisierung eines nicht-menschlichen Tiers mit einem Novirhabdovirus nach einem der Ansprüche 1 bis 6 umfasst, das das antigene Protein von Interesse umfasst.

## Claims

1. Recombinant novirhabdovirus the genome of which, in addition to the genes encoding the endogenous N, P, M, G and L proteins of said novirhabdovirus, contains an exogenous gene coding for a chimeric protein, said chimeric protein being **characterized in that** it comprises the sequence of an antigenic protein of interest, said antigenic protein of interest not being a glycoprotein, and is fused at its N-terminal end with the peptide signal of the G protein of a rhabdovirus and at its C-terminal end with a fragment of sequence of a rhabdovirus G protein, said fragment comprising the transmembrane domain of said rhabdovirus G protein or a portion thereof comprising at least 15 consecutive C-terminal amino acids of said domain, **characterized in that**, following after the transmembrane domain or the portion thereof, it comprises the intravirion domain of a rhabdovirus G protein, or a portion of said intravirion domain comprising at least 3 consecutive N-terminal amino acids of said domain.

2. The recombinant novirhabdovirus according to claim 1, **characterized in that** said transmembrane domain and/or said intravirion domain is (are) selected from among those of the G proteins of lyssavirus, vesiculovirus and novirhabdovirus.

3. The recombinant novirhabdovirus according to any of claims 1 or 2, **characterized in that** it is devoid of the endogenous NV gene, and **in that** the gene coding for the chimeric protein is inserted to replace said NV gene.

4. The recombinant novirhabdovirusaccording to any of claims 1 to 3, **characterized in that** it contains the endogenous NV gene, and **in that** the gene coding for the chimeric protein is inserted in an additional transcriptional unit placed in an intergenic region of the viral genome.

5. The recombinant novirhabdovirus according to claim 4, **characterized in that** it contains at least two additional transcriptional units each of which contains an exogenous gene coding for a chimeric protein such as defined in one of claims 1 or 2.

6. The recombinant novirhabdovirus according to any of claims 1 to 5, **characterized in that** it is selected from among a virus of infectious haematopoietic necrosis, and a virus of viral haemorrhagic septicaemia.

7. cDNA molecule of the genome of a recombinant novirhabdovirus according to any of claims 1 to 6.

8. The recombinant novirhabdovirus according any of claims 1 to 6 for use as vaccine inducing an immune response directed against the antigen of interest expressed by said novirhabdovirus.

9. The recombinant novirhabdovirus according to any of claims 1 to 6 for use as vaccine according to claim 8, **characterized in that** said vaccine is selected from among an anti-viral vaccine, anti-bacterial vaccine, anti-fungal vaccine, anti-parasite vaccine or anti-tumour vaccine.

10. Non-therapeutic method for producing antibodies directed against an antigenic protein of interest, said method comprising the immunisation of a non-human animal with a novirhabdovirus according to any of claims 1 to 6 comprising said antigenic protein of interest.


### SEQUENCE LISTING

<110> INSTITUT NATIONAL DE LA RECHERCHE AGRONOMIQUE BREMONT, Michel NZONZA, Angella
<120> NOVIRHABDOVIRUS RECOMBINANT UTILISABLE COMME VECTEUR D'ANTIGÈNES
<130> MJP-11-F539-159WO
<150> 1259815
   <151> 2012-10-15
<160> 5
<170> PatentIn version 3.5
<210> 1
   <211> 94
   <212> DNA
   <213> Artificial Sequence
<220>
   <223> PCR primer
<400> 1
   actagtatgg acaccacgat caccactccg ctcattctca ttctgatcac ctgcggagca 60
   gctagcggaa caacctatgg cgtctgttca aagg 94
<210> 2
   <211> 56
   <212> DNA
   <213> Artificial Sequence
<220>
   <223> PCR primer
<400> 2
   ggcccctccc acaaccccca tcccagataa cgctcctttg agggtggttg taaagg 56
<210> 3
   <211> 56
   <212> DNA
   <213> Artificial Sequence
<220>
   <223> PCR primer
<400> 3
   cctttacaac caccctcaaa ggagcgttat ctgggatggg ggttgtggga ggggcc 56
<210> 4
   <211> 34
   <212> DNA
   <213> Artificial Sequence
<220>
   <223> PCR primer
<400> 4
   tacgtatcag accgtctgac ttctagagaa ctgc 34
<210> 5
   <211> 31
   <212> DNA
   <213> Artificial Sequence
<220>
   <223> PCR primer
<400> 5
   actagtatgg acaccacgat caccactccg c 31